# EUROPEAN PATENT APPLICATION

(11) **EP 4 786 965 A1**
(43) Date of publication of application: **05.08.2026**
(21) Application number: 24871490.9
(22) Date of filing: 29.07.2024
(51) Int. Cl.: G01N 27/12, G01N 27/04

(54) **GAS SENSOR**

(30) Priority: 27.09.2023 JP 2023166128; 11.06.2024 JP 2024094150
(71) Applicant: Niterra Co., Ltd., Nagoya-shi, Aichi 461-0005 (JP)
(72) Inventor: KATO Tomofumi, Nagoya-shi, Aichi 461-0005 (JP); IKAWA Koichi, Nagoya-shi, Aichi 461-0005 (JP); KONDO Tomonori, Nagoya-shi, Aichi 461-0005 (JP); TAKAKURA Masahiro, Nagoya-shi, Aichi 461-0005 (JP); IGARASHI Kenta, Nagoya-shi, Aichi 461-0005 (JP)
(74) Representative: Diehl & Partner
(86) International application number: PCT/JP2024/026999
(87) International publication number: WO 2025/069688

(57) **Abstract**

A gas sensor 100 includes a stacked body 11 in which electrode layers 31 and 32L and an insulating layer 24L alternatingly stacked, and a sensitive membrane 40 which reacts with a gas under measurement. The stacked body 11 has a concave portion 11A1 which is concave in a stacking direction. The concave portion 11A1 has a side surface 11C including end surfaces of the electrode layers 31 and 32L and an end surface of the insulating layer 24L. The sensitive membrane 40 is in contact with a portion of the side surface 11C of the concave portion 11A1, the portion corresponding to one insulating layer 24L and at least one pair of electrode layers 31 and 32L sandwiching the one insulating layer 24L.

## Description

### TECHNICAL FIELD

The present disclosure relates to a gas sensor.

### BACKGROUND ART

Conventionally, as a technique relating to a sensor, the technique described in Patent Literature 1 has been known. Specifically, Patent Literature 1 discloses a sensor in which a first Au layer is formed on a silicon substrate, and a second Au layer is formed to be separated from the first Au layer by using Al₂O₃, whereby a nanogap whose size corresponds to the thickness of Al₂O₃ is formed. When a solution is injected to the nanogap of the sensor, the sensor can detect a particular substance through a change in an electrical property between opposite ends of the nanogap.

### CITATION LIST

### PATENT LITERATURE

Patent Literature 1: JP2006-234799A

### SUMMARY OF INVENTION

### TECHNICAL PROBLEM

It is desired for the configuration disclosed in Patent Literature 1 to improve its sensitivity of detecting the particular substance. A conceivable measure for improving the detection sensitivity is to provide a sensitive layer (sensitive membrane) in the sensor, in which the sensitive layer is in contact with, for example, two electrode layers (metal layers) and an insulating layer therebetween and reacts with the particular substance. However, in such a case, the sensitive layer may be bent or have a concave portion and/or a convex portion to follow the shapes of the electrode layers and the insulating layer, which brings about a risk of occurrence of cracking or rupturing (this is called step breakage) at this concave or convex portion or a like portion.

The present disclosure discloses a technique which has been completed on the basis of the above-described circumstances, and one object is to provide a gas sensor which can improve the sensitivity of detection. Another object is to provide a gas sensor which can suppress the step breakage.

### SOLUTION TO PROBLEM

A gas sensor of the present disclosure comprises a stacked body including electrode layers and an insulating layer alternatingly stacked, and a sensitive membrane which reacts with a gas under measurement, wherein the stacked body has a concave portion which is concave in a stacking direction, wherein the concave portion has a side surface including end surfaces of the electrode layer and an end surface of the insulating layer, and wherein the sensitive membrane is in contact with a portion of the side surface of the concave portion, the portion corresponding to one insulating layer and at least one pair of electrode layers sandwiching the one insulating layer.

### ADVANTAGEOUS EFFECT OF INVENTION

According to the present disclosure, it becomes possible to provide a gas sensor which can improve the sensitivity of detection. In addition, it becomes possible to provide a gas sensor which can suppress the step breakage.

### BRIEF DESCRIPTION OF DRAWINGS

[FIG. 1] Plan view schematically representing a gas sensor device according to Embodiment 1.
[FIG. 2] View showing the sectional configuration of a portion of a gas sensor.
[FIG. 3] Sectional view showing, on an enlarged scale, a sloping surface of a stacked body on one side and its vicinity.
[FIG. 4] Plan view showing, on an enlarged scale, a portion of the front surface of the stacked body.
[FIG. 5] Plan view schematically representing a gas sensor device according to Embodiment 2.
[FIG. 6] View showing the sectional configuration of a portion of a gas sensor.
[FIG. 7] Plan view showing a concave portion according to Embodiment 3.
[FIG. 8] Plan view showing a concave portion according to Embodiment 4.
[FIG. 9] Plan view showing a concave portion according to Embodiment 5.

### DESCRIPTION OF EMBODIMENTS

First, embodiments of the present disclosure will be listed and described.
(1) A gas sensor of the present disclosure comprises a stacked body including electrode layers and an insulating layer alternatingly stacked, and a sensitive membrane which reacts with a gas under measurement, wherein the stacked body has a concave portion which is concave in a stacking direction, wherein the concave portion has a side surface including end surfaces of the electrode layer and an end surface of the insulating layer, and wherein the sensitive membrane is in contact with a portion of the side surface of the concave portion, the portion corresponding to one insulating layer and at least one pair of electrode layers sandwiching the one insulating layer.
(2) In the gas sensor described in (1), the side surface of the concave portion may be inclined in relation to the stacking direction.
(3) In the gas sensor described in (1) or (2), the concave portion may have a bottom surface formed by a front surface of the electrode layer perpendicular to the stacking direction, wherein the sensitive membrane is in contact with the bottom surface of the concave portion.
(4) In the gas sensor described in any of (1) to (3), an outline of the concave portion may have a regular hexagonal shape, a circular shape, an equilateral triangular shape, or a rhombic shape in a plan view.
(5) In the gas sensor described in any of (1) to (4), a plurality of the concave portions may be disposed such that their density becomes the maximum in a plan view.

### <Embodiment 1>

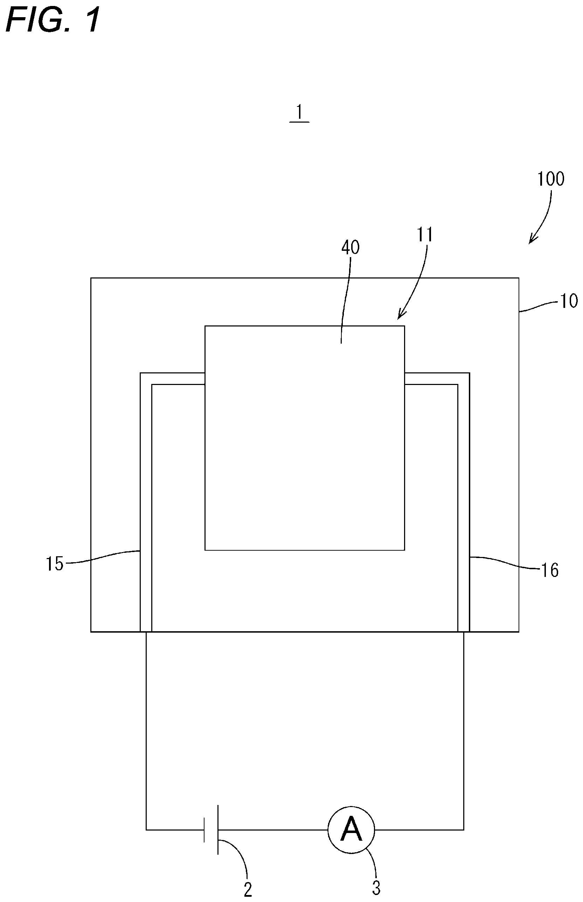

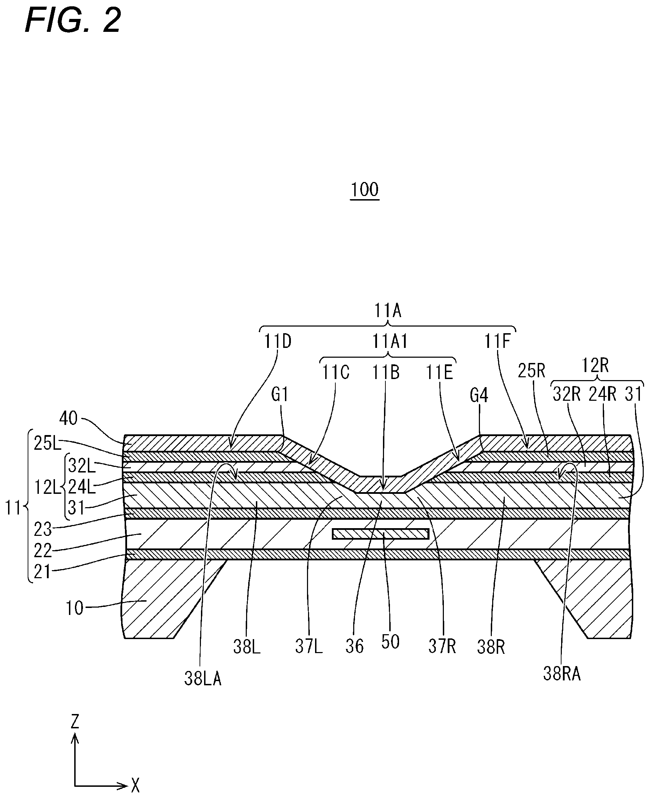

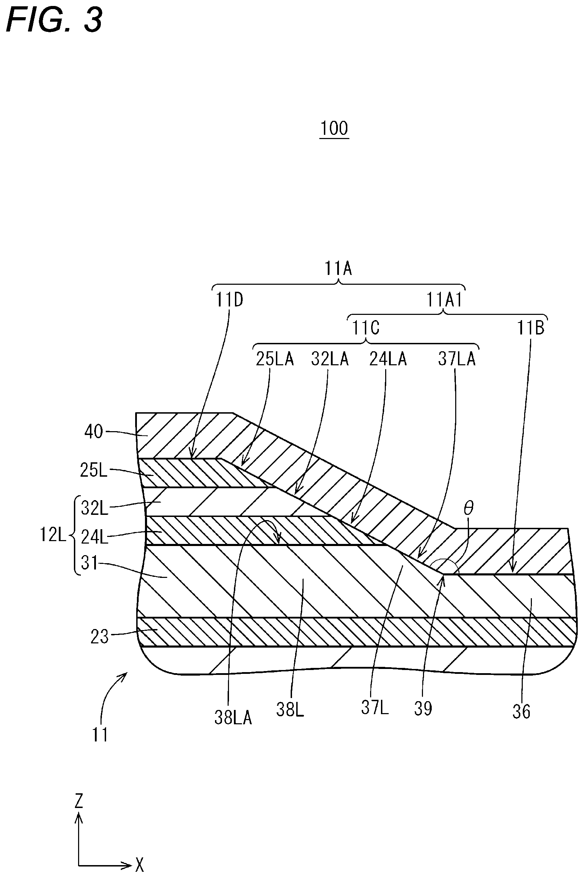

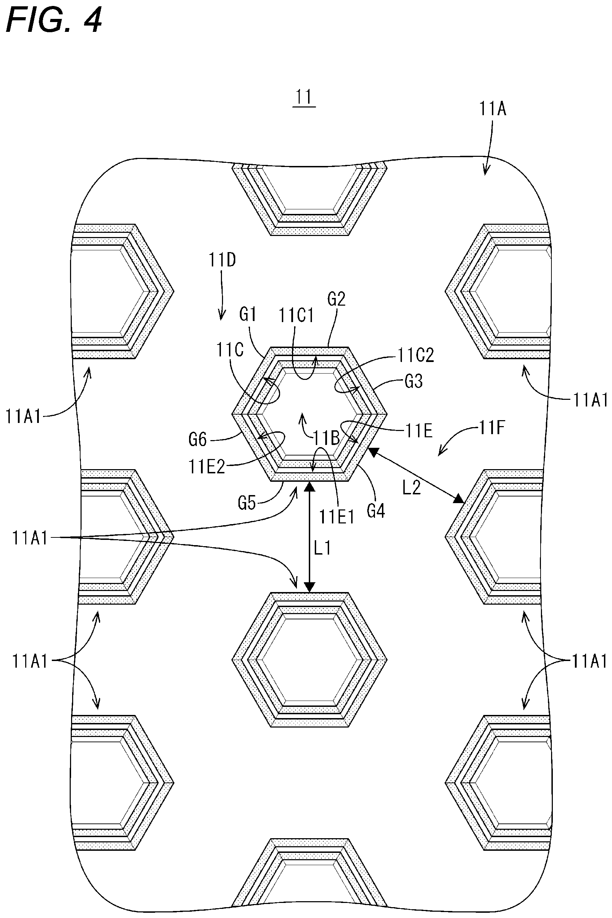

Embodiment 1 of the present disclosure will be described with reference to FIG. 1, etc. In the present embodiment, a gas sensor 100 which can detect a particular gas (gas under measurement) is shown as an example. Notably, in FIGS. 2 and 3, a Z direction corresponds to a stacking direction. and an X direction perpendicularly intersecting the Z direction corresponds to a horizontal direction. In addition, in each of FIGS. 1 and 4, the near side of the sheet corresponds to the front surface side in the gas sensor 100 (the upper side in the stacking direction), and, in each of FIGS. 2 and 3, the upper side of the sheet corresponds to the front surface side in the gas sensor 100 (the upper side in the stacking direction). In addition, in each of FIGS. 1 and 4, the far side of the sheet corresponds to the back surface side in the gas sensor 100 (the lower side in the stacking direction), and, in each of FIGS. 2 and 3, the lower side of the sheet corresponds to the back surface side in the gas sensor 100 (the lower side in the stacking direction).

As shown in FIG. 1, the gas sensor 100 includes a substrate 10 having a flat-platelike shape, a stacked body 11 stacked on the front surface of the substrate 10, and first and second wiring conductors 15 and 16 disposed on the front surface of the substrate 10 and connected to the stacked body 11. The substrate 10 is made of, for example, silicon. The gas sensor 100 constitutes a gas sensor device 1 together with a power supply 2 and an ammeter 3. The power supply 2 is connected to the first wiring conductor 15 and the ammeter 3. The ammeter 3 is connected to the second wiring conductor 16.

As shown in FIG. 2, the stacked body 11 includes a plurality of electrode layers 31, 32L, and 32R and a plurality of insulating layers 21, 22, 23, 24L, 24R, 25L, and 25R which are stacked in the stacking direction (vertical direction). Specifically, the first insulating layer 21, the second insulating layer 22, and the third insulating layer 23 are stacked in this order on the front surface of the substrate 10. The first electrode layer 31, the fourth insulating layers 24L and 24R, the second electrode layers 32L and 32R, and the fifth insulating layers 25L and 25R are stacked in this order on the front surface of the third insulating layer 23. As to the layers from the third insulating layer 23 to the fifth insulating layers 25L and 25R, the plurality of insulating layers 23, 24L, 24R, 25L, and 25R and the plurality of electrode layers 31, 32L, and 32R are alternatingly stacked in the stacking direction.

The fourth insulating layers 24L and 24R, the second electrode layers 32L and 32R, and the fifth insulating layers 25L and 25R include one-side layers 24L, 32L, and 25L disposed on the left side of the sheet in relation to a first horizontal surface 11B, which will be described later, and other-side layers 24R, 32R, and 25R disposed on the right side of the sheet in relation to the first horizontal surface 11B. The first electrode layer 31, the fourth insulating layer 24L on the left side, and the second electrode layer 32L on the left side will be collectively referred to as a one-side main body layer portion 12L. In addition, the first electrode layer 31, the fourth insulating layer 24R on the right side, and the second electrode layer 32R on the right side will be collectively referred to as an other-side main body layer portion 12R.

In the stacking direction, each of the distance between the first electrode layer 31 and the left-side second electrode layer 32L (the thickness of the left-side fourth insulating layer 24L) and the distance between the first electrode layer 31 and the right-side second electrode layer 32R (the thickness of the right-side fourth insulating layer 24R) is on the order of nanometer. These distances may be, for example, 1 nm or greater and less than 1000 nm, may be less than 100 nm, or may be several tens nm. The first wiring conductor 15 shown in FIG. 1 is connected to the first electrode layer 31. The second wiring conductor 16 is connected to each of the second electrode layers 32L and 32R.

No particular limitation is imposed on the materials of the electrode layers 31, 32L, 32R and the wiring conductors 15 and 16. Any material having electrical conductivity can be used. For example, of metals such as aluminum, copper, gold, platinum, etc., one or two or more metals can be employed. No particular limitation is imposed on the materials of the insulating layers 21, 22, 23, 24L, 24R, 25L, and 25R. Any material which does not have electrical conductivity can be used. Compounds such as SiO₂, Si₃N₄, etc. can be employed. A heater 50 which can heat the stacked body 11 is provided in the second insulating layer 22.

The front surface 11A of the stacked body 11 has a first horizontal surface 11B which extends perpendicularly to the stacking direction (in the horizontal direction), second horizontal surfaces 11D and 11F which extend perpendicularly to the stacking direction and are located on the upper side in relation to the first horizontal surface 11B, and sloping surfaces 11C and 11E which are continuous with the first horizontal surface 11B and with the second horizontal surfaces 11D and 11F and are inclined in relation to the stacking direction. The first horizontal surface 11B and the sloping surfaces 11C and 11E constitute a concave portion 11A1 in the stacked body 11. The concave portion 11A1 is concave in the stacking direction in relation to the second horizontal surfaces 11D and 11F. The sloping surfaces 11C and 11E and the second horizontal surfaces 11D and 11F include one-side surfaces 11C and 11D located on the left side of the sheet in relation to the first horizontal surface 11B and other-side surfaces 11E and 11F located on the right side of the sheet in relation to the first horizontal surface 11B.

At the left-side sloping surface (side surface) 11C of the concave portion 11A1, the first electrode layer 31 and the left-side second electrode layer 32L serve as a pair of electrode layers which sandwich the left-side fourth insulating layer (one insulating layer) 24L. Similarly, at the right-side sloping surface (side surface) 11E of the concave portion 11A1, the first electrode layer 31 and the right-side second electrode layer 32R serve as a pair of electrode layers which sandwich the right-side fourth insulating layer (one insulating layer) 24R.

The first electrode layer 31 has a thin layer portion 36, thick layer portions 38R and 38L thicker than the thin layer portion 36, and gradually changing layer portions 37R and 37L which are located between the thin layer portion 36 and the thick layer portions 38R and 38L and whose thicknesses change gradually in the horizontal direction. The thick layer portions 38R and 38L are thicker than the second electrode layers 32R and 32L. The gradually changing layer portions 37R and 37L and the thick layer portions 38R and 38L include one-side layer portions 37L and 38L located on the left side of the sheet in relation to the thin layer portion 36 and other-side layer portions 37R and 38R located on the right side of the sheet in relation to the thin layer portion 36. The left-side fourth insulating layer 24L is stacked on the front surface 38LA of the left-side thick layer portion 38L. Similarly, the right-side fourth insulating layer 24R is laid on the front surface 38RA of the right-side thick layer portion 38R.

The first horizontal surface 11B is the front surface of the first electrode layer 31 perpendicular to the stacking direction and is the front surface (upper surface) of the thin layer portion 36. The first horizontal surface 11B is also a bottom surface which constitutes the bottom of the concave portion 11A1. The second horizontal surfaces 11D and 11F are the front surfaces (upper surfaces) of the fifth insulating layers 25L and 25R.

The sloping surfaces 11C and 11E are side surfaces of the concave portion 11A1 and are inclined in relation to the stacking direction. As shown in FIG. 3, the one-side sloping surface 11C is formed by a continuous series of side surfaces; i.e., a side surface 37LA of the left-side gradually changing layer portion 37L (a side surface of the first electrode layer 31), a side surface 24LA of the left-side fourth insulating layer 24L, a side surface 32LA of the left-side second electrode layer 32L, and a side surface 25LA of the left-side fifth insulating layer 25. The side surfaces 37LA, 24LA, 32LA, and 25LA of these layers will be referred to as "end surfaces" in some cases. The left-side side surface (the sloping surface 11C) of the concave portion 11A1 includes the end surfaces 37LA and 32LA of the electrode layers (the first electrode layer 31 and the left-side second electrode layer 32L) and the end surface 24LA of the insulating layer (the left-side fourth insulating layer 24L). These end surfaces 37LA, 24LA, and 32LA constitute a side surface (end surface) of the one-side main body layer portion 12L.

The first electrode layer 31 is a particular electrode layer which is the lowest layer in the main body layer portions 12L and 12R and has the first horizontal surface 11B continuous with the sloping surfaces 11C and 11E. The first electrode layer 31, which is the particular electrode layer, has the side surface 37LA which is the side surface 37LA of the left-side gradually changing layer portion 37L, extends obliquely upward from the first horizontal surface 11B of the first electrode layer 31, and constitutes a portion of the one-side sloping surface 11C. The angle θ of a corner portion 39 between the first horizontal surface 11B and the one-side sloping surface 11C (or the side surface 37LA) is an obtuse angle greater than 90 degrees. The other-side sloping surface 11E has the same configuration as the above-described configuration of the one-side sloping surface 11C (symmetrical).

As shown in FIGS. 2 and 3, the gas sensor 100 includes a sensitive membrane 40 which extends over and is in contact with the bottom surface 11B of the concave portion 11A1, the side surfaces 11C and 11E of the concave portion 11A1, and the surfaces (the second horizontal surfaces 11D and 11F) located outside the concave portion 11A1. The sensitive membrane 40 extends over and is in contact with the entirety of the front surface 11A of the stacked body 11. At the side surfaces 11C and 11E of the concave portion 11A1, the sensitive membrane 40 is in contact with the side surface 24LA of one insulating layer 24L (or 24R) and the side surfaces 37LA and 32LA of at least a pair of electrode layers 31 and 32L (or 31 and 32R) which sandwich the one insulating layer 24L (or 24R). The sensitive membrane 40 extends from the first horizontal surface 11B to the side surface 37LA of the left-side gradually changing layer portion 37L (a portion of the one-side sloping surface 11C which is continuous with the first horizontal surface 11B) and is in contact therewith. The sensitive membrane 40 is in contact with the corner portion 39. Moreover, the sensitive membrane 40 extends from the one-side sloping surface 11C to the one-side second horizontal surface 11D and is in contact therewith. The other-side sloping surface 11E has the same configuration as the configuration described above (symmetrical).

In FIG. 4, an enlarged plan view of a portion of the front surface 11A of the stacked body 11 is shown, and the sensitive membrane 40 is not shown. The concave portion 11A1 has a regular hexagonal outline in the plan view. Specifically, the concave portion 11A1 has a bottom surface 11B having a regular hexagonal shape in the plan view and six sloping surfaces 11C, 11C1, 11C2, 11E, 11E1, and 11E2 which respectively start from six end portions of the bottom surface 11B and extend obliquely in relation to the stacking direction. The sloping surfaces 11C and 11E are located at the opposite ends (on the opposite sides) of the bottom surface 11B and form a pair. The sloping surfaces 11C1 and 11E1 are located at the opposite ends (on the opposite sides) of the bottom surface 11B and form a pair. The sloping surfaces 11C2 and 11E2 are located at the opposite ends (on the opposite sides) of the bottom surface 11B and form a pair. Respective upper end portions G1, G2, G3, G4, G5, and G6 of the sloping surfaces 11C, 11C1, 11C2, 11E, 11E1, and 11E2 define the outline of the concave portion 11A1 and form a regular hexagonal shape as a whole in the plan view.

On the front surface 11A of the stacked body 11, a plurality of concave portions 11A1 are disposed such that their density in the plan view becomes the maximum. In the present embodiment, the plurality of concave portions 11A1 are disposed in a honeycomb-like pattern in the plan view. Specifically, the plurality of concave portions 11A1 are disposed such that the distance between a certain concave portion 11A1 and another concave portion 11A1 located adjacent thereto becomes equal to the distance between the certain concave portion 11A1 and still another concave portion 11A1 located adjacent to the certain concave portion 11A1. For example, the distance L1 between a certain concave portion 11A1 located slightly above the center of the sheet and another concave portion 11A1 located below the certain concave portion 11A1 (i.e., located slightly below the center of the sheet) is equal to the distance L2 between the certain concave portion 11A1 located slightly above the center of the sheet and still another concave portion 11A1 located lower right in relation to the certain concave portion 11A1 (i.e., located rightward of the center of the sheet).

Notably, the sensitive membrane 40 extends over and is in contact with the plurality of concave portions 11A1 and the surfaces (the second horizontal surfaces 11D and 11F, etc.) therebetween. In addition, in each concave portion 11A1, the sensitive membrane 40 extends over and is in contact with the first horizontal surface 11B and the sloping surfaces 11C, 11C1, 11C2, 11E, 11E1, and 11E2.

The sensitive membrane 40 is configured such that it reacts with a particular gas (gas under measurement) and its electrical characteristic (for example, resistance) changes accordingly. No particular limitation is imposed on the material of the sensitive membrane 40 so long as the material is appropriately selected in accordance with a gas to be detected. For example, an oxide semiconductor film can be employed. The oxide semiconductor film may be formed of any of ZnO, SnO₂, WO₃, In₂O₃, TiO₂, V₂O₅, etc. When, in the gas sensor 100, the particular gas reacts with the sensitive membrane 40 and the resistance of the sensitive membrane 40 changes, in the gas sensor device 1, the current value detected by the ammeter 3 connected to the power supply 2 changes. Thus, the gas sensor device 1 can detect the particular gas.

Notably, no particular limitation is imposed on a method of manufacturing the gas sensor 100. For example, in a step of forming the stacked body 11, as shown in FIG. 2, the sloping surfaces 11C and 11E, etc. and the first horizontal surface 11B may be formed by performing ion etching from the front surface side of the fifth insulating layers 25L and 25R.

Next, the effect of the present embodiment will be described. The gas sensor 100 of the present embodiment includes the stacked body 11 in which the electrode layers 31 and 32L and the insulating layer 24L are alternatingly stacked, and the sensitive membrane 40 which reacts with the gas under measurement. The concave portion 11A1, which is concave in the stacking direction, is formed on the stacked body 11. The concave portion 11A1 has the side surface 11C including the end surfaces of the electrode layers 31 and 32L and the end surface of the insulating layer 24L. The sensitive membrane 40 is in contact with a portion of the side surface 11C of the concave portion 11A1, the portion corresponding to one insulating layer 24L and at least a pair of electrode layers 31 and 32L which sandwich the one insulating layer.

In such a gas sensor 100, the pair of electrode layers 31 and 32L are insulated by the one insulating layer 24R, and the sensitive membrane 40 is in contact with the pair of electrode layers 31 and 32L. The gas under measurement reacts with the sensitive membrane 40, and, in the sensitive membrane 40, for example, its electrical characteristic changes. Thus, detection of the gas under measurement through the two electrode layers 31 and 32L can be performed with high sensitivity and high selectivity. In addition, since the sensitive membrane 40 is in contact with a portion of the side surface 11C of the concave portion 11A1, the portion corresponding to one insulating layer 24L and at least a pair of electrode layers 31 and 32L which sandwich the one insulating layer, the area of contact with the sensitive membrane 40 can be increased, and an anchor effect can be obtained. Thus, the detection sensitivity of the gas sensor 100 can be improved. For example, in the case where the plurality of electrode layers 31 and 32L are disposed on the front surface of the insulating layer 24R and the sensitive membrane 40 is in contact with the electrode layers 31 and 32L, the sensitive membrane 40 has concave and convex portions to follow the shapes of the electrode layers 31 and 32L, etc., and step breakage may occur at the concave and convex portions. In contrast, in the above-described gas sensor 100, since the sensitive membrane 40 is in contact with the pair of electrode layers 31 and 32L and the side surface 11C of the concave portion 11A1 including the end surfaces of the electrode layers 31 and 32L and the end surface of the insulating layer 24R, it is possible to avoid formation of the above-described concave and convex portions on the sensitive membrane 40. Thus, it is possible to suppress the step breakage.

The side surface 11C of the concave portion 11A1 is inclined in relation to the stacking direction.

In such a gas sensor 100, since the area of contact with the sensitive membrane 40 can be increased as compared with the case where the side surface of the concave portion is not inclined and extends in the stacking direction (the case where the side surface of the concave portion is perpendicular to the front surface of the electrode layer), the detection sensitivity can be improved. In addition, it is possible to avoid formation of the sensitive membrane 40 in contact with the side surface 11C of the concave portion 11A1 and the electrode layers 31 and 32L such that the sensitive membrane 40 is bent at right angle. Thus, it is possible to suppress the step breakage.

The concave portion 11A1 has the bottom surface 11B, which is constituted by the front surface of the electrode layer 31 which is perpendicular to the stacking direction, and the sensitive membrane 40 is in contact with the bottom surface 11B of the concave portion 11A1.

In such a gas sensor 100, it is possible to increase the area of contact between the electrode layers 31 and 32L and the sensitive membrane 40, thereby improving the detection sensitivity.

The concave portion 11A1 has a regular hexagonal outline in the plan view.

In such a gas sensor 100, the side surfaces 11C of the concave portion 11A1, which define the sides of the regular hexagonal outline of the concave portion 11A1 in the plan view, are in contact with the sensitive membrane 40 over the same distance (area). Therefore, current is more likely to flow equally at each side.

A plurality of concave portions 11A1 are disposed such that their density in the plan view becomes the maximum.

In such a gas sensor 100, when electricity is applied to the electrode layers 31 and 32L, current is more likely to flow equally to each concave portion 11A1. In addition, since a plurality of concave portions 11A1 are disposed in a staggered arrangement, a plurality of concave portions 11A1 can be disposed appropriately in one gas sensor 100, and the area of contact with the sensitive membrane 40 can be increased.

### <Embodiment 2>

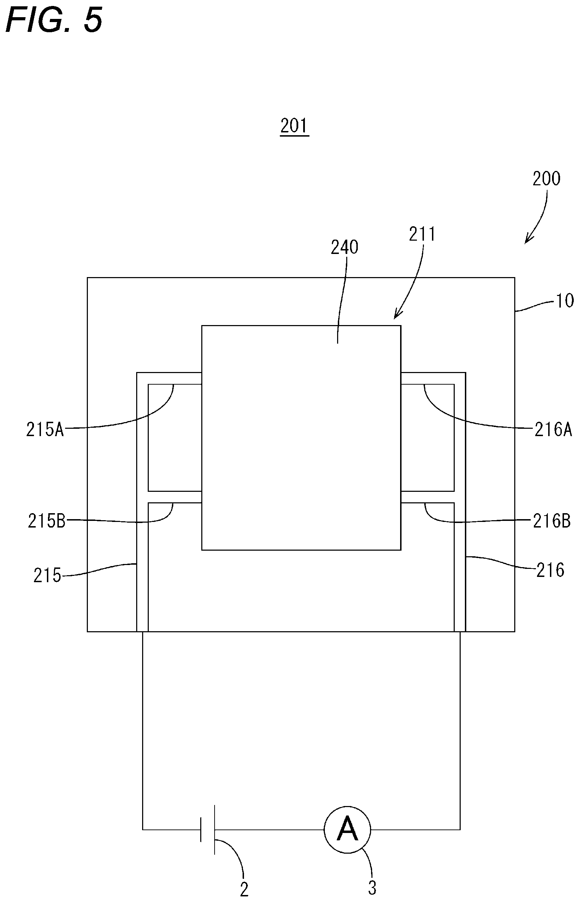

Next, Embodiment 2 of the present disclosure will be described with reference to FIG. 5, etc. In the present embodiment, portions identical to those of the above-described embodiment are denoted by the same signs, and, as to its structure, action, and effect, their redundant descriptions are omitted.

As shown in FIG. 5, a gas sensor 200 includes a first wiring conductor 215 and a second wiring conductor 216 connected to a stacked body 211. The first wiring conductor 215 is branched into a far-side wiring conductor 215A and a near-side wiring conductor 215B. Similarly, the second wiring conductor 216 is branched into a far-side wiring conductor 216A and a near-side wiring conductor 216B. The gas sensor 200 constitutes a gas sensor device 201 together with the power supply 2 and the ammeter 3.

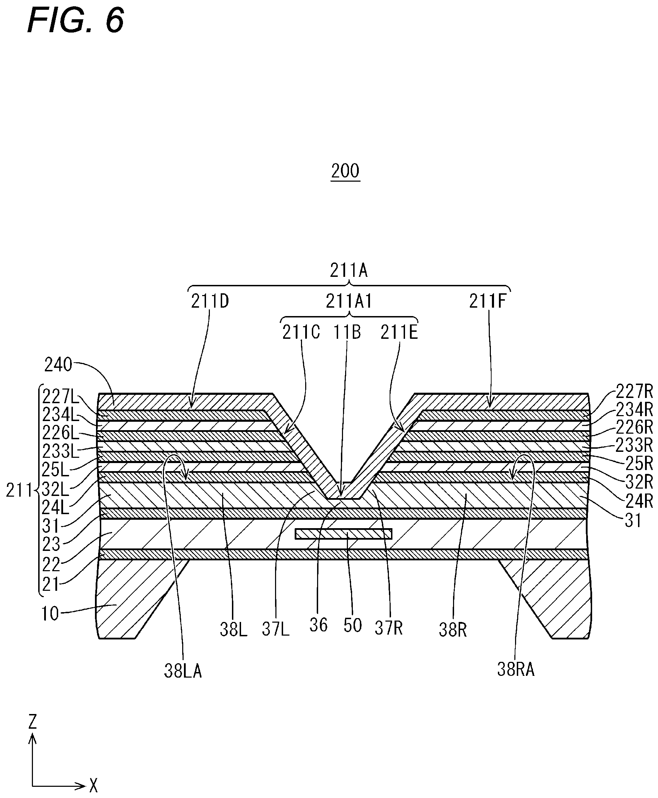

As shown in FIG. 6, the stacked body 211 includes a plurality of electrode layers 31, 32L, 32R, 233L, 233R, 234L and 234R and a plurality of insulating layers 21, 22, 23, 24L, 24R, 25L, 25R, 226L, 226R, 227L, and 227R, which are stacked in the stacking direction (vertical direction). The left-side third electrode layer 233L, the left-side sixth insulating layer 226L, the left-side fourth electrode layer 234L, and the left-side seventh insulating layer 227L are stacked in this order on the front surface of the left-side fifth insulating layer 25L. The right-side third electrode layer 233R, the right-side sixth insulating layer 226R, the right-side fourth electrode layer 234R, and the right-side seventh insulating layer 227R are stacked in this order on the front surface of the right-side fifth insulating layer 25R.

The far-side wiring conductor 215A of the first wiring conductor 215 shown in FIG. 1 is connected to the first electrode layer 31. The far-side wiring conductor 216A of the second wiring conductor 216 is connected to the second electrode layers 32L and 32R. The near-side wiring conductor 215B of the first wiring conductor 215 is connected to the third electrode layers 233L and 233R. The near-side wiring conductor 216B of the second wiring conductor 216 is connected to the fourth electrode layers 234L and 234R.

On the front surface 221A of the stacked body 211, the first horizontal surface 11B is the front surface (upper surface) of the thin layer portion 36. A sloping surface 211C on one side is formed by a continuous series of side surfaces; i.e., the side surface of the gradually changing layer portion 37L, the side surface of the fourth insulating layer 24L, the side surface of the second electrode layer 32L, the side surface of the fifth insulating layer 25L, the side surface of the third electrode layer 233L, the side surface of the sixth insulating layer 226L, the side surface of the fourth electrode layer 234L, and the side surface of the seventh insulating layer 227L. A sloping surface 211E on the other side has the same configuration as the sloping surface 211C on the one side (symmetrical). The first horizontal surface 11B and the sloping surfaces 211C and 211E constitute a concave portion 211A1 in the stacked body 211. The concave portion 211A1 is concave in the stacking direction in relation to the second horizontal surfaces 211D and 211F. The second horizontal surfaces 211D and 211F are the front surfaces (upper surfaces) of the seventh insulating layers 227L and 227R. A sensitive membrane 240 extends from the first horizontal surface 211B to the sloping surfaces 211C and 211E and then to the second horizontal surfaces 211D and 211F and is contact with the first horizontal surface 211B, the sloping surfaces 211C and 211E, and the second horizontal surfaces 211D and 211F.

### <Embodiment 3>

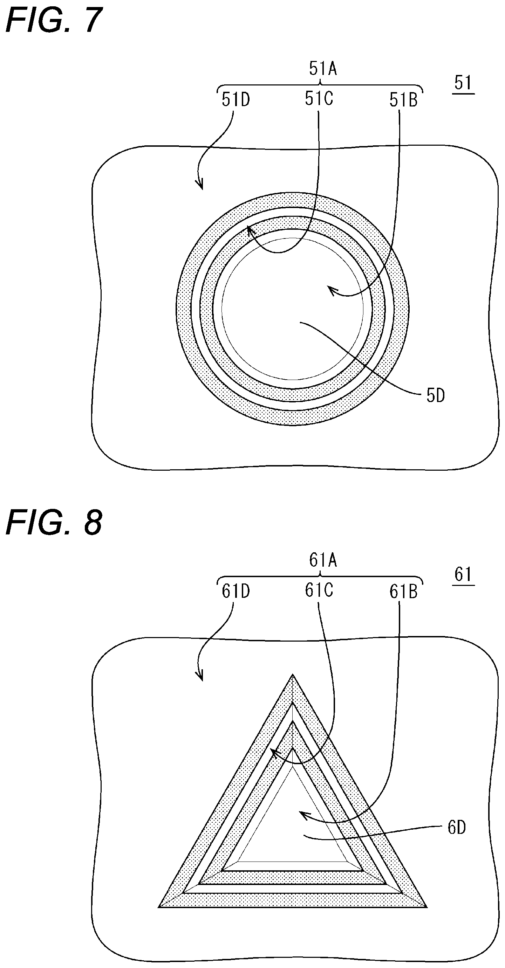

Next, Embodiment 3 of the present disclosure will be described with reference to FIG. 7. The present embodiment is one that is obtained by changing the shape of the front surface of the stacked body 11 of Embodiment 1. Portions corresponding to those of Embodiment 1 are denoted by signs each obtained by changing the value at the tens place of the numeral portion of a corresponding sign from 1 to 5, and, as to its structure, action, and effect, their redundant descriptions are omitted.

As shown in FIG. 7, the front surface 51A of a stacked body 51 has a first horizontal surface 51B which is a surface extending perpendicular to the stacking direction (in the horizontal direction), a second horizontal surface 51D located above the first horizontal surface 51B, and a sloping surface 51C which is a surface inclined in relation to the stacking direction. A concave portion 5D with a bottom is formed by the sloping surface 51C and the second horizontal surface 51B. The outline of the concave portion 5D has a circular shape in the plan view.

### <Embodiment 4>

Next, Embodiment 4 of the present disclosure will be described with reference to FIG. 8. The present embodiment is one that is obtained by changing the shape of the front surface of the stacked body 11 of Embodiment 1. Portions corresponding to those of Embodiment 1 are denoted by signs each obtained by changing the value at the tens place of the numeral portion of a corresponding sign from 1 to 6, and, as to its structure, action, and effect, their redundant descriptions are omitted.

As shown in FIG. 8, the front surface 61A of a stacked body 61 has a first horizontal surface 61B which is a surface extending perpendicular to the stacking direction (in the horizontal direction), a second horizontal surface 61D located above the first horizontal surface 61B, and a sloping surface 61C which is a surface inclined in relation to the stacking direction. A concave portion 6D with a bottom is formed by the sloping surface 61C and the second horizontal surface 61B. The outline of the concave portion 6D has an equilateral triangular shape in the plan view.

### <Embodiment 5>

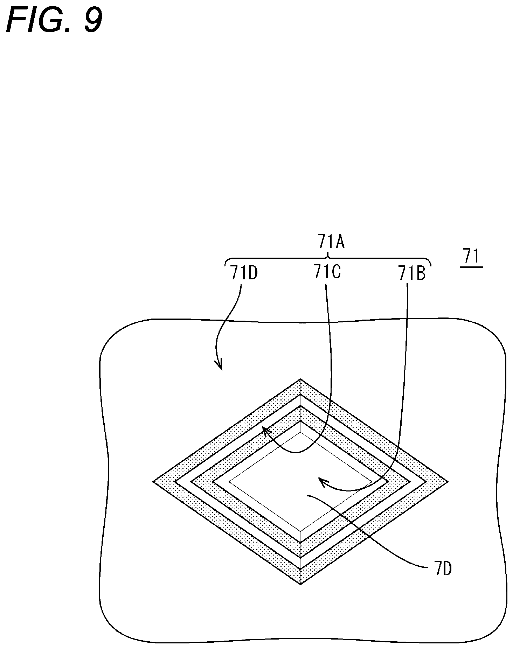

Next, Embodiment 5 of the present disclosure will be described with reference to FIG. 9. The present embodiment is one that is obtained by changing the shape of the front surface of the stacked body 11 of Embodiment 1. Portions corresponding to those of Embodiment 1 are denoted by signs each obtained by changing the value at the tens place of the numeral portion of a corresponding sign from 1 to 7, and, as to its structure, action, and effect, their redundant descriptions are omitted.

As shown in FIG. 9, the front surface 71A of a stacked body 71 has a first horizontal surface 71B which is a surface extending perpendicular to the stacking direction (in the horizontal direction), a second horizontal surface 71D located above the first horizontal surface 71B, and a sloping surface 71C which is a surface inclined in relation to the stacking direction. A concave portion 7D with a bottom is formed by the sloping surface 71C and the second horizontal surface 71B. The outline of the concave portion 7D has a rhombic shape in the plan view.

### <Other embodiments>

The present disclosure is not limited to the embodiments described by the above description and the drawings, and, for example, the following embodiments fall within the technical scope of the present disclosure. Moreover, in addition to the following modifications, various modifications may be employed for implementation of the technique of the present disclosure without departing from the gist.
(1) The configuration of the stacked body can be changed appropriately from the configurations employed in the above-described embodiments. It is sufficient that the stacked body includes at least a first electrode layer, an insulating layer stacked on the first electrode layer, and a second electrode layer stacked on the insulating layer. In such a configuration, the upper surface of the second electrode layer may be in contact with a sensitive membrane.
(2) The configuration of the sensitive membrane can be changed appropriately from the configurations employed in the above-described embodiments. The sensitive membrane may be configured such that it is in contact with the first horizontal surface and the sloping surface and is not in contact with the second horizontal surface. Alternatively, the sensitive membrane may be configured such that it is in contact with a portion of the first horizontal surface and the sloping surface which is continuous with the portion of the first horizontal surface.
(3) The configuration of the concave portion can be changed appropriately. The side surface (sloping surface) of the concave portion may extend in a direction along the stacking direction. The shape of the outline of the concave portion in the plan view is not limited to the regular hexagonal shape, the equilateral triangular shape, the rhombic shape, and the circular shape and may be any of other triangular shapes, rectangular shapes, and other polygonal shapes. In addition, the stacked body may be configured such that a plurality of concave portions whose outlines extend in one direction are arranged on the front surface of the stacked body such that the concave portions have a slit-like shape in the plan view.
(4) The configuration of the wiring conductors can be changed appropriately. In the above-described embodiments, the wiring conductors are routed to be connected to the sides of the stacked body located on opposite sides in the lateral direction (the left side and right side of the sheet in FIG. 1). However, the routing is not limited thereto. For example, the wiring conductors may be routed to be connected to the side of the stacked body located on the near side (the lower side of the sheet in FIG. 1).
(5) The instrument used for measuring the electrical characteristics in the gas sensor device is not limited to an ammeter for measuring current, and measuring instruments for measuring various electrical characteristics such as a voltmeter and an ohmmeter may be employed.

### REFERENCE SIGNS LIST

11, 211: stacked body, 11A1, 211A1: concave portion, 11B: first horizontal surface (bottom surface), 11C, 11E, 211C, 211E: sloping surface (side surface), 24L, 24R, 224L, 224R: fourth insulating layer (one insulating layer), 31: first electrode layer, 32L, 32R: second electrode layer, 40, 240: sensitive membrane, 100, 200: gas sensor

## Claims

1. A gas sensor comprising a stacked body including electrode layers and an insulating layer alternatingly stacked, and a sensitive membrane which reacts with a gas under measurement,
wherein the stacked body has a concave portion which is concave in a stacking direction,
wherein the concave portion has a side surface including end surfaces of the electrode layer and an end surface of the insulating layer, and
wherein the sensitive membrane is in contact with a portion of the side surface of the concave portion, the portion corresponding to one insulating layer and at least one pair of electrode layers sandwiching the one insulating layer.

2. The gas sensor according to claim 1, wherein the side surface of the concave portion is inclined in relation to the stacking direction.

3. The gas sensor according to claim 1 or 2, wherein the concave portion has a bottom surface formed by a front surface of the electrode layer perpendicular to the stacking direction, and
wherein the sensitive membrane is in contact with the bottom surface of the concave portion.

4. The gas sensor according to claim 1 or 2, wherein an outline of the concave portion has a regular hexagonal shape, a circular shape, an equilateral triangular shape, or a rhombic shape in a plan view.

5. The gas sensor according to claim 1 or 2, wherein a plurality of the concave portions are disposed such that their density becomes the maximum in a plan view.
